# EUROPEAN PATENT SPECIFICATION

(11) **EP 2 836 146 B1**
(45) Date of publication and mention of the grant of the patent: **20.07.2016**
(21) Application number: 13724866.2
(22) Date of filing: 10.04.2013
(51) Int. Cl.: A61B 17/34

(54) **Dual channel surgical device for abdomen access**
Chirurgische Doppelkanalvorrichtung für Abdomenzugang
Dispositif chirurgical à double canal pour un accès à l'abdomen

(30) Priority: 11.04.2012 IT BA20120020 U
(43) Date of publication of application: 18.02.2015
(73) Proprietor: Nuzziello, Vincenzo, 71122 Foggia (IT)
(72) Inventor: Nuzziello, Vincenzo, 71122 Foggia (IT)
(74) Representative: Russo, Dimitri
(86) International application number: PCT/IB2013/000649
(87) International publication number: WO 2013/153432

(56) References cited:
- EP-A2- 1 593 348
- EP-A2- 2 238 932
- WO-A1-2012/005819
- WO-A2-2010/041900
- US-A1- 2010 249 516

## Description

The present patent concerns a medical/surgical device, called *trocar,* used for laparoscopic surgery.

Laparoscopic surgery is a surgical technique in which operations are performed without laparotomy but through the use of a camera connected to a monitor and surgical instruments such as scissors, clamps, electrocoagulators, staplers, needle holders, etc., which are introduced through small incisions into the abdominal wall. To this purpose, a gas, usually carbon dioxide or nitrogen protoxide, is first injected into the abdominal cavity in order to create the necessary space for manipulations of the surgical instruments. Subsequently, a camera is introduced and, under direct visual control, the other trocars are introduced to provide for the insertion of the surgical instruments.

In particular, the device according to the present invention as defined in independent claim 1, consists of two channels, in other words it has two cannulas, parallel to one another and capable of accommodating, at the same time, a camera and a surgical instrument or two surgical instruments.

As known, in order to perform laparoscopic surgery the patient must not have previously undergone surgery using the same technique.

The main obstacle is represented by the presence of postoperative adhesions, which sometimes constitute an insurmountable wall. Mastery and constant use of the laparoscopic technique has increasingly expanded the applications range, including the approach to previously operated abdomens.

The main difficulty remains, however, the entry and the limited view in suitable areas to obtain sufficient triangulation to perform any procedure. In these cases, it is therefore necessary to open a way amongst the adhesions to create a sufficiently wide space to introduce the trocars.

Another problem is represented by the increasingly frequent use of diagnostic laparoscopy valid and useful especially in the staging of abdominal neoplasms. In these cases, the reduced invasiveness and the better overall vision, along with the better clinical results and a greater appreciation for the procedure by the patient, constitute significant advantages.

Surgical devices for laparoscopic surgery are known from the prior art. As an example, document No. US 2010/0249516, which forms the basis of the preamble of claim 1, discloses a surgical device including a flexible tissue retractor having an outer surface for engaging a body incision, an internal surface defining a passageway, a sleeve whose at least a portion is positionable within the passageway, an insert comprising at least one opening, adapted to be releasably supported in the passageway. Said device is used exclusively for enabling access into the abdomen through the umbilical incision not allowing a lateral access to the abdomen, which would permit an easier access to the surgical site. In addition, the surgical device provides for four accesses to the body so that the surgical instruments cannot work in parallel and will interfere with one another.

Another example of known trocar can be found in EP No. 1593348 used exclusively for neurological surgery.

The purpose of this patent is to solve the problems associated with laparoscopic surgery, ensuring better efficiency, greater safety, minimally invasive procedure and maximal utility, responding to the current surgical trend requiring access through one umbilical incision, otherwise called *"Single portfor two".*

A further purpose is that of equipping the jacket for the insertion of the trocar with a two-way movable valve which not only is an excellent seal of the pneumoperitoneum, but which can be removed, if necessary, in case there is the need to quickly empty the abdomen or to remove important anatomical pieces from this access.

A further purpose is that of providing a trocar through which a camera and a grasping or cutting instrument can be accommodated contemporaneously provided that they do not create an obstacle to each other.

A yet further purpose is that of allowing, through a better placement of taps for the introduction of the CO2, the elimination of the edges from the main supporting body allowing, as a consequence, a greater range of motion to the surgeon.

Finally, the trocar aims at reducing as much as possible damage to the patient's internal organs during its introduction into the abdomen, unlike what happens with the introduction of trocars equipped with blade.

These and other objectives of the present patent will be illustrated through the following detailed description of the preferred embodiment, which is not intended to limit the scope of the patent, with reference to the attached drawings:
Fig. 1 is an exploded axonometric view of the dual channel surgical device in a preferred embodiment;
Fig. 2 is an axonometric view of the surgical device of Fig. 1 fully assembled;
Fig. 3 is a side view, long side, of the three main elements of the trocar disassembled, of the supporting body;

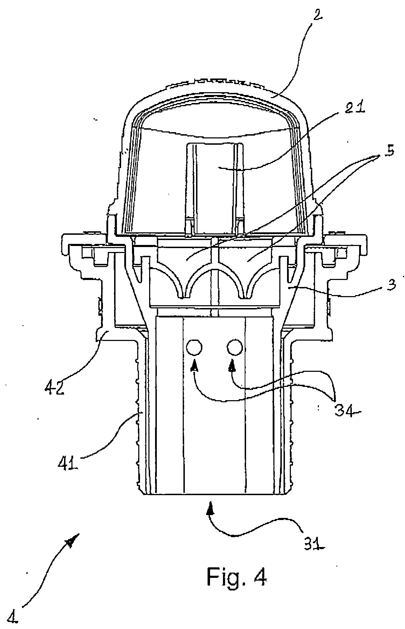

Fig. 4 is a section view of the trocar;
Fig. 5 is an exploded axonometric view of the insert including the two-way valve and the valve block;
Fig. 6 is a top view of the supporting body;
Fig. 7 is a side view, long side, of the supporting body of Fig. 6;
Fig. 8 is a side view, short side, of the supporting body of Fig. 6 and Fig. 7;
Fig. 9 is a bottom view of the supporting body of Fig. 6, 7 and 8;
Fig. 10 is an axonometric view of the extractor's seal block;
Fig. 11 is a sectional side view of the extractor;
Fig. 12 is a side view of the extractor.

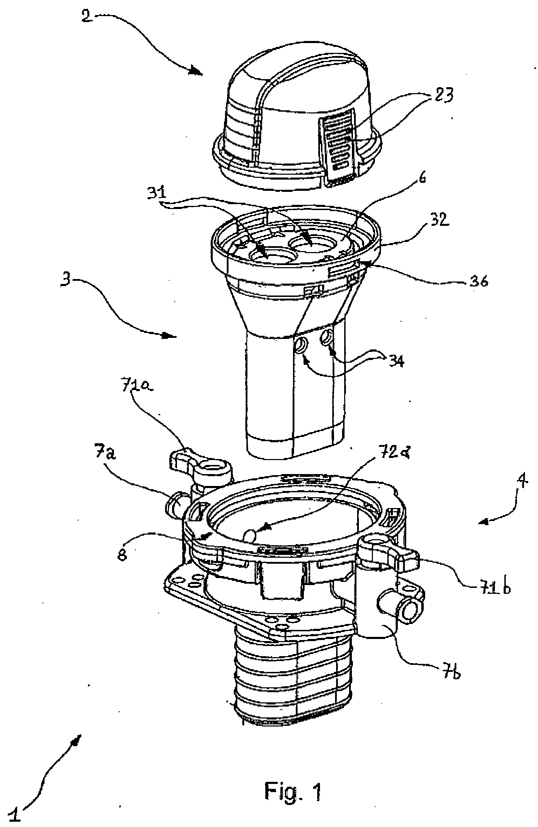

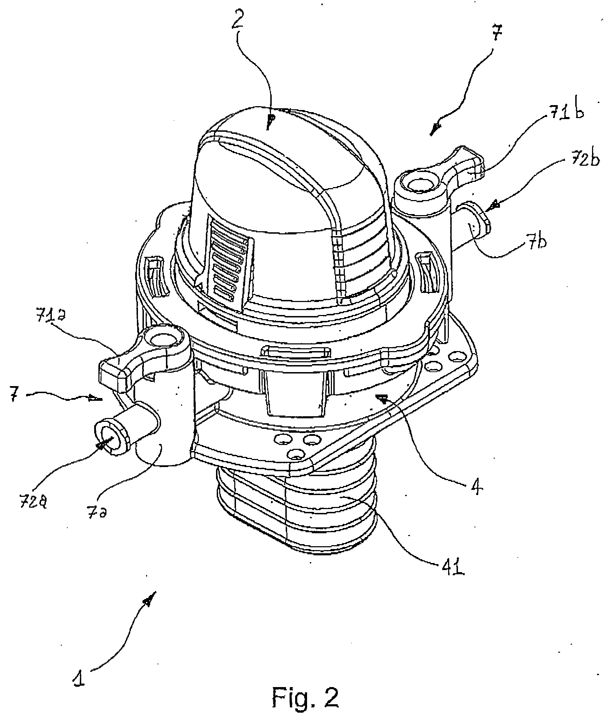

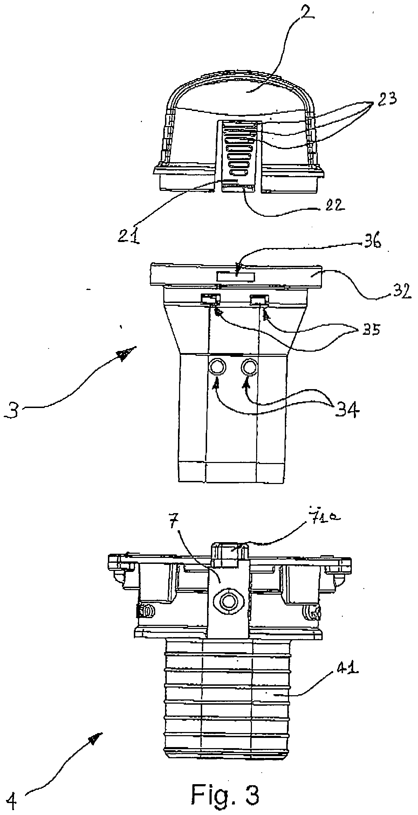

With reference to Figures 1, 2 and 3, the surgical device according to the present patent, identified as 1, consists of three main elements: an extractor 2, an insert3 with two cannulas 31, and a supporting body 4 with incorporated taps 7.

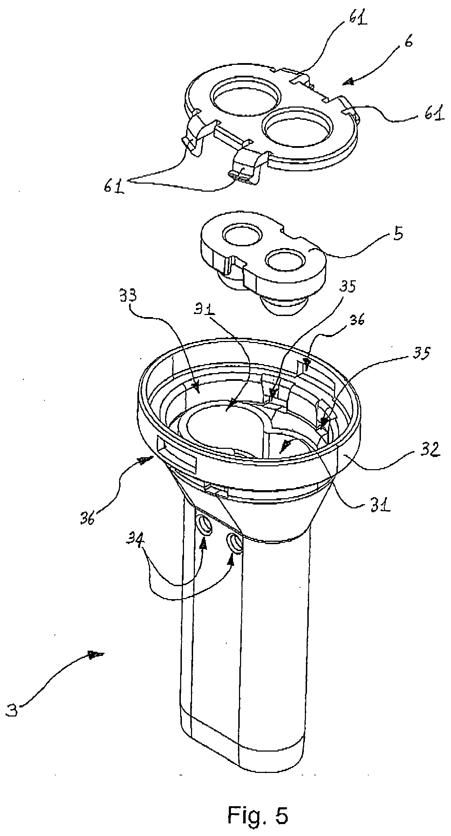

With reference to Figure 5, the main element of the surgical device is the insert 3 including two cannulas 31 parallel to each other and of equal length and internal diameter, fused together and capable of simultaneously receiving a camera and a grasping or cutting instrument required for the surgery.

The upper opening 33 of the insert 3 has the shape of a funnel 32 and incorporates a silicone rubber two-way movable valve 5 securely fastened to the insert 3 through a valve block 6, equipped, to this purpose, with four fixing teeth 61 fitting in as many seats 35 formed inside the opening 33.

Fastening of the valve block 6 through the aforesaid teeth 61 provides the possibility to remove the two-way movable valve 5, if necessary, to remove important anatomical pieces.

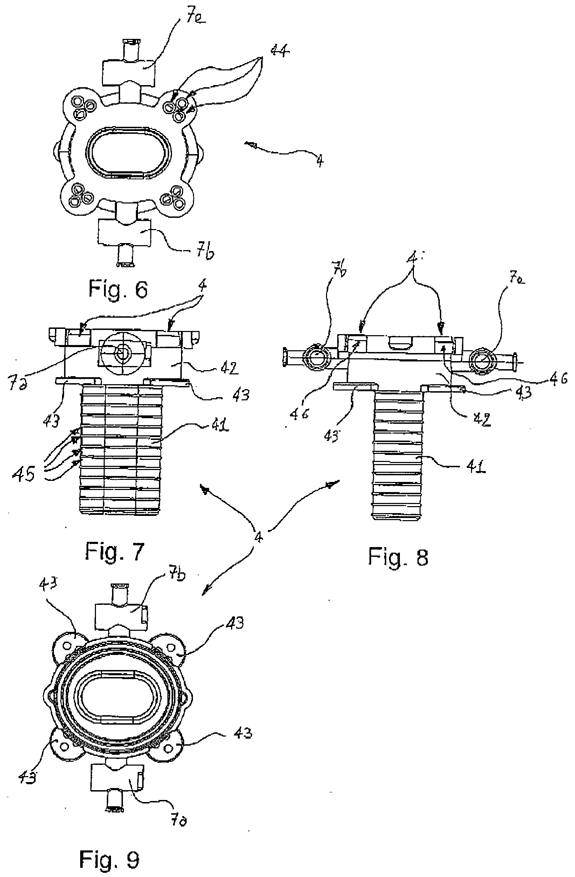

In particular, with reference to Figures 6 to 9, a further important element of the surgical device 1 is the body 4 which, together with the insert 3 aims at opening the abdomen, as well as at guiding the insertion of the surgical instruments into the patient's abdomen.

Said body 4 is formed of an oval tube 41, a head 42, two taps 7 incorporated in said head 42, stabilizing fins 43 to stabilize the same body 4 after its introduction into the patient's body. Three holes 44 are provided on said fins 43, to allow the passage of sutures.

Four protrusions 46 are provided outside the head 42 of the body 4 to engage with four elastic teeth 81 perpendicularly positioned to the seal block 8.

The adoption of a fastening by means of the elastic teeth 81 makes the seal block 8 removable from the body 4.

In order to increase the pneumatic seal of the body 4, the outer surface of the oval tube 41 is provided with a series of seal rings 11 which keep the CO2 from leaking from the patient's body.

As mentioned, the body 4 is provided with two taps 7 wherein the tap 7a injects and maintains, at the right pressure, the CO2 or another gas into the abdominal cavity for the creation of the pneumoperitoneum and the tap 7b for its discharge.

Said taps 7, and in particular the control levers 71, are rotated of 90° with respect to the head of the body 4, making their movement more efficient and natural.

The gas is introduced into the body 4 through a nozzle 72a, its flow is adjusted by the control lever 71a, and it is introduced inside the cannulas 31 through the holes 34. Otherwise, the gas escapes from the body 4 through the nozzle 72b, and its flow is regulated by the control lever 71b.

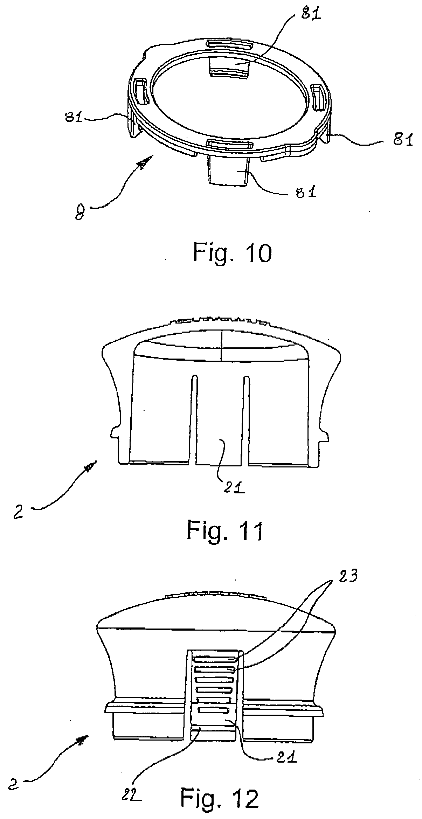

With reference to Figures 11 and 12, the device 1 is equipped with an extractor 2 comprising a pair of elastic elements 21 provided with teeth 22 at their ends. Said teeth are suitable for engaging with the notches 36 formed on the upper edge of the funnel 32 of the insert 3.

In order to allow an easier extraction of the insert 3 from the body 4, the extractor 2 is provided with a knurling 23.

In order to perform laparoscopic surgery, a pneumoperitoneum is first created by the surgeon. This is achieved by inflating the peritoneal cavity with sterile, heated and humidified carbon dioxide to obtain elevation and separation of the abdominal wall from the internal organs. Insufflation may be performed using a Veress needle (with a retractable protection mechanism) which allows the gas necessary to create the pneumoperitoneum to access the peritoneal cavity through a periumbilical incision.

The Veress needle is then extracted and the body 4 is inserted into the same incision, the body 4 being directly connected to the insufflating device through the tap 7a in order to ensure sufficient Insufflation to compensate loss of gas during introduction of the instruments. The same procedure can be performed directly making a cut into the site and entering the body 4 without the aid of the Veress needle, thereby introducing the CO2.

Subsequently, after insertion of the insert 3 inside the body 4, the abdominal cavity is reached. It is now possible to introduce the surgical instruments such as the camera to display the cavity and the other surgical instruments.

The main indication of the device is adhesiolysis in previously operated abdomens with laparoscopic technique or with traditional surgery, allowing exploration and removal of the adhesions through a single incision.

Further therapeutic indications are diagnostic and bioptic exploration, staging, cholecystectomy, appendicectomy, hepatic access, ovarian cysts draining out, ovariectomy etc.

## Claims

1. Dual channel surgical device for abdomen access comprising an extractor (2), an insert (3) and a supporting body (4), wherein the insert (3) includes two cannulas (31) of equal length and diameter, fused together into a single piece and placed within said insert (3), and wherein said insert (3) is of a size such as to enable its insertion inside said supporting body (4), **characterized in that** said insert (3) comprises a funnelled proximal end (32), and that said insert (3) can be removed from the supporting body (4) through an extractor (2), said extractor (2) being equipped with elastic elements (21) having teeth (22) at their terminal ends, said teeth (22) being suitable to engage with the insert (3) through notches (36) at the upper edge of the insert's funnelled proximal end (32).

2. Surgical device according to claim 1, **characterised in that** the extractor (2) is provided with a knurling (23), which is a non-slip surface.

3. Surgical device according to one or more of the preceding claims, **characterized in that** said supporting body (4) is formed of an oval tube (41), a head (42), two taps (7) incorporated in said head (42), and stabilizing fins (43).

4. Surgical device according to claims 1 and 3, **characterized in that** said stabilizing fins (43) are transverse to the head (42) of the body (4), on which body (4) one or more holes (44) are formed.

5. Surgical device according to claims 1, 3 and 4, **characterized in that** on two opposite sides of the head (42) two taps (7) are placed, wherein the control levers (71) of said taps (7) are rotated by 90 ° with respect to the head (42) of said body (4).

6. Surgical device according to claims 1, 3, 4 and 5, **characterized in that** on the outer surface of the oval tube (41) a series of seal rings (45) are placed.

7. Surgical device according to one or more of the preceding claims, **characterized in that** within the upper funnel-shaped opening (33) of the insert (3) a movable silicone rubber two-way valve (5) is placed, said valve (5) being blocked, when needed, by a valve block (6), said valve block (6) being equipped with four teeth (61) to elastically engage with seats (35) formed inside the opening (33).

## Patentansprüche

1. Chirurgisches Zweikanal-Gerät für den Zugang zum Bauchraum, bestehend aus einem Absaugapparat (2), einem Einsatz (3) und einem Stützkörper (4), wobei der Einsatz (3) zwei Kanülen (31) gleicher Länge und gleichen Durchmessers umfasst, die zusammen in ein einziges Stück eingeschmolzen und innerhalb des besagten Einsatzes (3) positioniert werden, und wobei der besagte Einsatz (3) eine solche Größe aufweist, die dessen Einschieben in den besagten Stützkörper (4) ermöglicht, **dadurch gekennzeichnet, dass** der besagte Einsatz ein trichterförmiges, proximales Ende (32) umfasst und dass der besagte Einsatz (3) aus dem Stützkörper (4) über einen Absaugapparat (2) entfernt werden kann, wobei der besagte Absaugapparat (2) mit elastischen Elementen (21) mit Zähnen (22) an ihren Terminalenden ausgestattet ist, wobei die besagten Zähne (22) geeignet sind, über Kerben (36) an der Oberkante des trichterförmigen, proximalen Endes (32) des Einsatzes in den Einsatz (3) zu greifen.

2. Chirurgisches Gerät gemäß Anspruch 1, **dadurch gekennzeichnet, dass** der Absaugapparat (2) mit einer Rändelung (23) versehen ist, die eine rutschfeste Oberfläche ist.

3. Chirurgisches Gerät gemäß einem oder mehreren der vorangehenden Ansprüche, **dadurch gekennzeichnet, dass** der Stützkörper (4) aus einem ovalen Rohr (41), einem Kopf (42), zwei Wasserhähnen (7), die in den besagten Kopf (42) integriert sind, und Stabilisierungsflossen (43) besteht.

4. Chirurgisches Gerät gemäß den Ansprüchen 1 und 3, **dadurch gekennzeichnet, dass** die besagten Stabilisierungsflossen (43) quer zum Kopf (42) des Körpers (4) verlaufen, wobei eine oder mehrere Öffnungen (44) an solchem Körper (4) ausgebildet werden.

5. Chirurgisches Gerät gemäß den Ansprüchen 1, 3 und 4, **dadurch gekennzeichnet, dass** auf zwei entgegengesetzten Seiten des Kopfes (42) zwei Wasserhähne (7) positioniert werden, wobei die Bedienungshebel (71) der besagten Wasserhähne (7) um 90 Grad in Bezug auf den Kopf (42) des besagten Körpers (4) gedreht werden.

6. Chirurgisches Gerät gemäß den Ansprüchen 1, 3, 4 und 5, **dadurch gekennzeichnet, dass** auf der Außenseite des ovalen Rohrs (41) eine Reihe von Dichtringen (45) vorgesehen ist.

7. Chirurgisches Gerät gemäß einem oder mehreren der vorangehenden Ansprüche, **dadurch gekennzeichnet, dass** innerhalb der oberen, trichterförmigen Öffnung (33) des Einsatzes (3) ein bewegliches Zwei-Wege-Ventil aus Silikonkautschuk (5) vorgesehen ist, wobei das besagte Ventil (5) bei Bedarf von einem Ventilblock (6) blockiert wird, wobei der besagte Ventilblock (6) mit vier Zähnen (61) ausgestattet ist, um elastisch in Sitze (35), die innerhalb dieser Öffnung (33) ausgebildet werden, zu greifen.

## Revendications

1. Instrument chirurgical à double canal pour l'accès à l'abdomen comprenant un extracteur (2), un insert (3) et un corps de support (4), dans lequel l'insert (3) comprend deux canules (31) de même longueur et diamètre, assemblées en une pièce unique et installées à l'intérieur de l'insert (3), et dans lequel l'insert (3) est d'une dimension telle à permettre son insertion dans ledit corps de support (4), **caractérisé par le fait que** cet insert (3) comprend une extrémité proximale évasée (32) et **le fait que** ledit insert (3) peut être retiré du corps de support (4) à l'aide d'un extracteur (2), ledit extracteur (2) équipé d'éléments élastiques (21) pourvus de dents (22) à leurs extrémités, ces dents (22) étant conçues pour s'insérer dans l'insert (3) à travers des encoches (36) pratiquées au niveau du bord supérieur de l'extrémité proximale évasée (32).

2. Instrument chirurgical selon la revendication 1, **caractérisé par le fait que** l'extracteur (2) est pourvu d'un moletage (23) constituant une surface antidérapante.

3. Instrument chirurgical conforme à une ou plusieurs des revendications précédentes, **caractérisé par le fait que** ledit corps de support (4) est constitué d'un tube ovale (41), d'une tête (42), de deux robinets (7) intégrés dans la tête (42) et de pattes de stabilisation (43).

4. Instrument chirurgical selon les revendications 1 et 3, **caractérisé par le fait que** lesdites pattes de stabilisation (43) sont orientées dans un sens transversal par rapport à la tête (42) du corps (4), dans lequel corps (4) un ou plusieurs trous (44) sont réalisés.

5. Instrument chirurgical selon les revendications 1, 3 et 4, **caractérisé par le fait que** duex robinets (7), dont les leviers de commande (71) sont orientés à 90° par rapport à la tête (42) du corps (4), ont été installés de part et d'autre de la tête (42).

6. Instrument chirurgical selon les revendications 1, 3, 4 et 5, **caractérisé par le fait que** la surface externe du tube ovale (41) est pourvue d'une série d'anneaux d'étanchéité (45).

7. Instrument chirurgical selon une ou plusieurs revendications précédentes, **caractérisé par le fait qu'**une vanne bidirectionnelle mobile en caoutchouc à la silicone (5) est installée à l'intérieur de l'ouverture supérieure évasée (33) de l'insert (3), ladite vanne (5) étant immobilisée, si nécessaire, par un verrou de vanne (6), ledit verrou de vanne (6) étant muni de quatre dents (61) s'insérant de manière élastique dans les logements (35) réalisés à l'intérieur de l'ouverture (33).
